# EUROPÄISCHE PATENTSCHRIFT

(11) **EP 2 904 977 B1**
(45) Veröffentlichungstag und Bekanntmachung des Hinweises auf die Patenterteilung: **28.11.2018**
(21) Anmeldenummer: 15153749.5
(22) Anmeldetag: 04.02.2015
(51) Int. Cl.: A61B 17/00, A61B 17/29

(54) **Chirurgisches Instrument und Handhabe für selbiges**
Surgical instrument and handle for same
Instrument chirurgical et poignée pour celui-ci

(30) Priorität: 10.02.2014 DE 102014101603
(43) Veröffentlichungstag der Anmeldung: 12.08.2015
(73) Patentinhaber: Karl Storz SE & Co. KG, 78532 Tuttlingen (DE)
(72) Erfinder: Merz, Robin, 78532 Tuttlingen (DE); Stefan, Jochen, 78532 Tuttlingen (DE)

(56) Entgegenhaltungen:
- EP-A2- 2 535 009
- WO-A-92/04059
- US-A1- 2002 026 202
- US-A1- 2003 009 176

## Beschreibung

Die nachfolgende Erfindung betrifft eine Handhabe für ein chirurgisches Instrument, das chirurgisches Instrument selbst sowie dessen Bedienverfahren und ein Verfahren zum Zerlegen des Instruments.

Chirurgische Instrumente zur Verwendung in der minimalinvasiven Chirurgie wie Scheren, Zangen, Nadelhalter und andere Greifer sowie Retraktoren sind bekannt. Das eigentliche Werkzeug bzw. der Werkzeugkopf ist dabei an einem distalen Ende eines Schafts angeordnet, wobei die Betätigung durch einen am proximalen Ende angeordneten Betätigungsgriff, der über eine Betätigungsstange oder einen Seilzug operativ mit dem Werkzeugkopf gekoppelt ist, erfolgt. Die Betätigung der Betätigungsstange kann dabei über ein Drehrad, das etwa über ein Bewegungsgewinde mit einem verschiebbaren Schlitten gekoppelt ist, realisiert sein. Mit dem Drehrad lässt sich jedoch nur eine Funktion des Werkzeugkopfes steuern, nämlich das Öffnen und Schließen in vorgegebenen Grenzen.

Um das chirurgische Instrument, besser reinigen zu können und um einen Betätigungsgriff mit verschiedenen Werkzeugköpfen einsetzen zu können, ist ferner bekannt, dass der Schaft abgekoppelt werden kann. Dafür sind an einem distalen Kopplungsabschnitt des Betätigungsgriffs und an dem verschiebbaren Antriebsschlitten Rastmittel angebracht, die von außen getrennt voneinander betätigt werden können, sodass zum Auslösen stets zwei Hände nötig sind Dokument WO9204059 A1 offenbart eine Handhabe mit zwei längsaxial verschiebbaren Bedienelementen, die zusammenwirken, um die Position eines Instrumentenschafts zu verriegeln. Ausgehend von diesem Stand der Technik liegt der vorliegenden Erfindung die Aufgabe zu Grunde, eine verbesserte Handhabe für ein chirurgisches Instrument zu schaffen, die es ermöglicht, eine zusätzliche Funktion eines Werkzeugkopfes zu steuern und die ergonomischer als bisher von einem Schaft entkoppelt werden kann.
Diese Aufgabe wird durch eine Handhabe für ein Instrument mit den Merkmalen des unabhängigen Anspruchs 1 gelöst.
Darüber hinaus ergibt sich die Aufgabe, ein chirurgisches Instrument zu schaffen, das ergonomischer als bekannte chirurgische Instrumente bedient werden kann und das leicht zerlegbar ist.
Diese Aufgabe wird durch ein chirurgisches Instrument mit den Merkmalen des unabhängigen Anspruchs 12 gelöst.
Bevorzugte Weiterbildungen der Vorrichtungen werden jeweils durch die Unteransprüche beschrieben.
Eine weitere Aufgabe der vorliegenden Erfindung ist es, ein Bedienverfahren für ein erfindungsgemäßes chirurgisches Instrument bereitzustellen, das es auf ergonomische und sichere Weise ermöglicht, die zusätzliche Funktion des Werkzeugkopfes zu bedienen.
Diese Aufgabe wird durch ein Bedienverfahren für ein chirurgisches Instrument mit den Merkmalen des Anspruchs 14 gelöst.
Schließlich ergibt sich noch die Aufgabe, ein Zerlegeverfahren für das erfindungsgemäße chirurgische Instrument zu schaffen, das es ermöglicht, das Instrument ergonomischer und schneller zu zerlegen.
Diese Aufgabe wird durch ein Zerlegeverfahren für ein chirurgisches Instrument mit den Merkmalen des Anspruchs 15 gelöst.

Die erfindungsgemäße Handhabe für ein chirurgisches Instrument umfasst in einer ersten Ausführungsform einen distalen Kopplungsabschnitt, der zur Kopplung mit einem Schaft, hierin immer ein Instrumentenschaft, vorgesehen ist. Die Handhabe umfasst ferner einen länglichen Grundkörper, in dem ein Schlitten längsaxial zwischen einer ersten Endposition und einer zweiten Endposition verschiebbar geführt ist. Länglich meint hier im Wesentlichen zylindrisch, wobei die Deckflächen des Zylinders, der ein Zylinder mit rundem, ovalem oder eckigen Querschnitt sein kann, nicht zwingend normal zur Längsachse sein müssen - in diesem Falle liegt eher ein röhrenförmiger Grundkörper vor. In beiden Fällen ist es so, dass der Grundkörper durchaus hohle und volle Längsachsenabschnitte haben kann. Die Handhabe weist ein Betätigungselement auf, das zur Erzeugung der Axialbewegung mit dem Schlitten operativ gekoppelt ist. Der Schlitten ist dazu vorgesehen, eine Betätigungsstange eines Schafts des chirurgischen Instruments zu bewegen. Die Handhabe hat ferner ein zweites Betätigungselement, das längsaxial benachbart zum ersten Betätigungselement an dem Grundkörper angeordnet ist, wobei das zweite Betätigungselement um die Längsachse des Grundkörpers drehbar und über diesem angeordnet ist. Darüber hinaus ist das zweite Betätigungselement wenigstens an seinem dem ersten Betätigungselement zugewandten Ende ringförmig und trägt an diesem Ende innenumfänglich einen Sperrring, der bezüglich des zweiten Betätigungselements drehfest ist. Eine Innenwand des Sperrrings hat eine radial in den Sperrring hinein weisende Auswölbung, die eine wenigstens zum Teil in der Ringebene liegende Fläche aufweist. Der Schlitten weist einen Finger auf, der sich von einer zum Sperrring weisenden Stirnfläche des Schlittens exzentrisch zur Längsachse des Grundkörpers erstreckt. In einem Sperrzustand des zweiten Betätigungselements stellt die Fläche der Auswölbung des Sperrrings einen ersten Anschlag für den Schlitten bereit, indem der Finger an der Fläche zur Anlage kommt. In einem Freigabezustand des zweiten Betätigungselements hingegen ist der Finger zur Fläche der Auswölbung des Sperrrings versetzt und gibt einen Verschiebewegabschnitt für den Schlitten frei.

Bei dem ersten Betätigungselement kann es sich insbesondere um ein Drehrad oder einen Hebel handeln, das / der bevorzugt koaxial auf dem Grundkörper geführt sein kann. Die Fläche der Auswölbung des Sperrrings kann vollständig in der Ringebene liegen, wobei es auch möglich ist, dass die Fläche unter einem Winkel zur Ringebene ausgerichtet ist, so dass nur eine gedachte Schnittgerade in der Ringebene liegt. Der Kopplungsabschnitt muss nicht ganz am äußeren distalen Ende der Handhabe angeordnet sein, sondern kann in einem distalen Endabschnitt vorliegen, wobei dieser Endabschnitt durchaus eine Länge, die der halben Gesamtlänge der Handhabe entspricht, aufweisen kann.

Der Verschiebewegabschnitt, der durch Verdrehen des zweiten Betätigungselements freigegeben wird, wird im Normalbetrieb der Handhabe nicht benötigt, vielmehr kann durch Verschieben des Schlittens entlang des zusätzlichen Verschiebewegabschnitts eine eher selten benötigte Funktion eines operativ mit der Handhabe gekoppelten chirurgischen Werkzeugs genutzt werden: So kann eine Zange oder Schere dadurch etwa in eine Überoffenstellung gebracht werden. Damit die Überoffenstellung nicht unbewusst erreicht wird, ist zunächst die willentliche Betätigung des zweiten Betätigungselements nötig. Vorteilhaft kann vorgesehen sein, dass die Winkelpositionen des zweiten Betätigungselements, die dem Sperrzustand respektive dem Freigabezustand entsprechen, durch entsprechende Anschläge vorgegeben sind, so dass die Sperrposition einfach durch Drehen in die eine Richtung und die Freigabeposition durch Drehen in die jeweilig andere Richtung erreicht werden kann, ohne dass darauf zu achten wäre, um welchen Winkelbetrag das zweite Betätigungselement verdreht wird. Als angularer Anschlag kann die Auswölbung selbst dienen, wobei lediglich ein der Freigabeposition und der Sperrposition entsprechender Gegenanschlag des Grundkörpers geschaffen werden muss. Je nach Einsatzgebiet der Handhabe kann das zweite Betätigungselement entweder proximal oder distal des ersten Betätigungselements angeordnet sein, wobei eine distale Anordnung vorteilhaft ist, da das erste Betätigungselement häufiger bedient wird als das zweite Betätigungselement, das lediglich zur Steuerung der "Zusatzfunktion" vorgesehen ist. Der Erfindungsgedanke der Zusammenwirkung des exzentrischen Fingers des Schlittens mit der wegdrehbaren Auswölbung des Sperrrings kann aber auch problemlos umgesetzt werden, wenn das zweite Betätigungselement proximal des ersten Betätigungselements angeordnet ist.

In einer weiteren Ausführungsform kann das zweite Betätigungselement entlang eines Längsachsenabschnitts des Grundkörpers verschiebbar sein. Über diesen Bewegungsfreiheitsgrad des zweiten Betätigungselements kann eine Funktion der Handhabe oder eine Funktion eines operativ mit der Handhabe gekoppelten chirurgischen Werkzeugs bedient werden.

In einer noch weiteren Ausführungsform kann der Grundkörper an seinem Außenumfang eine Nase aufweisen, die so positioniert ist, dass sie im Sperrzustand des zweiten Betätigungselements an der Fläche der Auswölbung des Sperrrings anliegt, während die Nase im Freigabezustand des zweiten Betätigungselements versetzt zur Auswölbung vorliegt. Alternativ oder zusätzlich kann die Auswölbung auch in Umfangrichtung des Sperrrings angeschrägt sein.

Indem die Abmessungen der Nase und der Auswölbung des Sperrrings aufeinander abgestimmt werden, kann der Bewegungsfreiheitsgrad entlang der Längsachse des Grundkörpers im Sperrzustand des zweiten Betätigungselements gesperrt werden und indem das zweite Betätigungselement in den Freigabezustand gebracht wird bei Bedarf freigegeben werden. Um die Funktion zu bedienen, die der längsaxialen Bewegung des zweiten Betätigungselements zugewiesen ist, muss das zweite Betätigungselement zunächst in den Freigabezustand rotiert werden. Erst im Freigabezustand des zweiten Betätigungselements kann der Sperrring zusammen mit dem zweiten Betätigungselement über die Nase geführt werden. Eine Anschrägung der Ausführung des Sperrrings ist vorteilhaft, da der Sperrring dadurch auch dann mit geringem Kraftaufwand gedreht werden kann, wenn der Finger des Schlittens draufgepresst wird, etwa durch starkes Betätigen des ersten Betätigungselements. Auch in Zusammenwirkung der Nase des Grundkörpers mit dem Finger des Schlittens muss sichergestellt sein, dass das zweite Betätigungselement mit dem Sperrring in seiner Freigabestellung längsaxial verschoben werden kann, so dass Finger und Nase gemeinsam durch die Ringebene treten können. Dazu müssen die Abmessungen und die Positionierung des Fingers und der Nase zusammen auf die Auswölbung des Sperrrings abgestimmt werden.

Die Nase kann insbesondere einen hakenförmigen Querschnitt aufweisen, der sich vorteilhaft im Wesentlichen entlang des Außenumfangs des Grundkörpers erstrecken kann, wobei ein Übergang des Grundkörpers zu der hakenförmigen Kontur der Nase bevorzugt tangential ausgestaltet sein kann. Darüber hinaus kann sich die Nase auch entlang eines Abschnitts parallel der Längsachse des Grundkörpers erstrecken. Die Nase schmiegt sich quasi als Fortsatz des Grundkörperquerschnitts an diesen an, sie ragt nicht weiter radial nach außen als der Grundkörper selbst, wodurch das zweite Betätigungselement problemlos auch bis über die Nase verschoben werden kann.

Ferner kann der Schlitten eine Kopplungsvorrichtung aufweisen, wobei eine Rastvorrichtung bevorzugt wird, die insbesondere ein Rastschieber sein kann und ein federbelasteter Rastschieber besonders bevorzugt wird, wobei die Betätigungsstange des Schafts lösbar mit der Kopplungsvorrichtung des Schlittens gekoppelt werden kann. Darüber hinaus kann auch der Grundkörper eine Kopplungsvorrichtung aufweisen, die eine Rastvorrichtung sein kann, wobei ein Rastschieber bevorzugt wird und ein federbelasteter Rastschieber besonders bevorzugt wird, wobei diese Kopplungsvorrichtung lösbar mit dem Hüllrohr des Schafts gekoppelt werden kann. Neben den letztgenannten Kopplungsvorrichtungen können auch andere Kopplungsvorrichtungen geeignet sein, die Erfindung wird jedenfalls hierdurch nicht beschränkt. Die Rastschieber können jeweils mit einem Betätigungsstift bzw. Betätigungspin verbunden sein, über die eine Betätigungskraft auf die Schieber ausgeübt werden kann. Der jeweilige Betätigungsstift oder Betätigungspin kann sich dazu insbesondere über die eine Außenkontur des Schlittens bzw. des Grundkörpers an der Längsachsenposition des Rastschiebers hinaus erstrecken.

Das zweite Betätigungselement kann des Weiteren operativ mit einer Auslösevorrichtung gekoppelt sein, die sich parallel zur Längsachse des Grundkörpers erstreckt. Die Auslösevorrichtung ist operativ mit der Kopplungsvorrichtung des Grundkörpers und des Schlittens verbunden und kann im Freigabezustand des zweiten Betätigungselements zusammen mit dem zweiten Betätigungselement längsaxial in eine Auslösestellung verschoben werden.

Durch den längsaxialen Verschiebefreiheitsgrad des zweiten Betätigungselements wird in dieser Ausführungsform ermöglicht, dass ein Schaft, der mit der Handhabe gekoppelt ist, auch wieder entkoppelt werden kann. Anders als bei bekannten Handhaben, bei denen stets zwei räumlich voneinander getrennte Rasten betätigt werden müssen, werden gemäß der vorliegenden Erfindung beide Rasten, die für die Betätigungsstange und die für das Hüllrohr des Schafts, durch eine gemeinsame Auslösevorrichtung ausgelöst. Die Auslösevorrichtung der vorliegenden Erfindung kann vorteilhaft auch nicht unbeabsichtigt betätigt werden, da der längsaxiale Bewegungsfreiheitsgrad des zweiten Betätigungselements lediglich im Freigabezustand des zweiten Betätigungselements aktiviert ist. Zusätzlich zu einer verbesserten Ergonomie beim Abkoppeln des Schafts bietet die erfindungsgemäße Handhabe eine hohe Sicherheit gegen Fehlbedienung.

Gemäß einer weiteren Ausführungsform kann die Auslösevorrichtung ein mit dem zweiten Betätigungselement mitbewegbares erstes Teil und ein in Bezug auf die Längsachse des Grundkörpers ortsfestes zweites Teil aufweisen. Der erste und / oder der zweite Teil der Auslösevorrichtung umfassen bevorzugt ein Auslöseband, das in einem oder mehreren Abschnitten im Längsschnitt keilförmige, wellenförmige oder angeschrägte Abschnitte aufweist. Die keilförmigen, wellenförmigen oder angeschrägten Abschnitte kontaktieren jeweils eine Auslöserippe des jeweils anderen Teils.

Ortsfest in Bezug zur Längsachse des Grundkörpers bedeutet hierin, dass eine Verschiebung des entsprechenden Teils der Auslösevorrichtung parallel zur Längsachse gesperrt ist jedoch eine Bewegung in andere Richtungen, insbesondere senkrecht zur Längsachse möglich ist. Der jeweilige keilförmige, wellenförmige oder angeschrägte Abschnitt gleitet bei Betätigung des längsaxialen Verschiebefreiheitsgrads des zweiten Betätigungselements auf der korrespondierenden Auslöserippe des andern Teils ab, wodurch eine Hubbewegungen der Auslösevorrichtung in einer Richtung senkrecht zur Verschieberichtung erzeugt wird. Diese Hubbewegung wird auf die Kopplungsvorrichtung des Schlittens und des Grundkörpers übertragen, wodurch der Eingriff des Hüllrohrs respektive der Betätigungsstange des Schafts mit den Kopplungsvorrichtungen gelöst wird. Das Auslöseband mit den keilförmigen, wellenförmigen oder angeschrägten Abschnitten kann der mitbewegte Teil oder der ortsfeste Teil sein. Es kann sogar vorgesehen sein, dass einer der beiden Teile integraler Bestandteil des Grundkörpers ist, so können die Auslöserippen etwa an den Grundkörper angeformt sein.

Zwischen dem zweiten Betätigungselement und dem Grundkörper kann des Weiteren eine Rückstellvorrichtung angeordnet sein, bei der es sich insbesondere um eine Feder handeln kann, die dazu vorgesehen ist, das zweite Betätigungselement zurückzustellen.

Die Ruhestellung, in die das zweite Betätigungselement zurückgestellt wird, ist dabei vorteilhaft die längsaxiale Position, in der die Ringebene des Sperrrings in einer Ebene mit einer zu dem Sperrring weisenden Stirnfläche der Nase weist, also eine Position, in der die Auslösevorrichtung unbetätigt ist. Dies stellt einen zusätzlichen Schutz gegen Fehlbedienung der Auslösevorrichtung dar. Zudem wird das zweite Betätigungselement stets durch die Rückstellvorrichtung stets in eine Längsachsenposition gebracht, in der nach dem Abkoppeln erneut ein Schaft angekoppelt werden kann.

Weiter kann der Grundkörper eine längsaxiale Führungsnut aufweisen, in der der Schlitten geführt ist. Befindet sich der Schlitten an seinem ersten Anschlag, so befindet sich zwischen der zu dem Sperrring weisenden Stirnfläche des Schlittens und einem gegenüberliegenden Ende der Führungsnut ein Abstand, der zumindest so groß ist wie die halbe Länge des Fingers. Hierdurch wird ermöglicht, dass der Verschiebewegabschnitt des Schlittens, der durch Verdrehung des Sperrrings zusätzlich freigegeben wird, begrenzt wird. Das Betätigungsband der Auslösevorrichtung kann sich in dieser Ausführungsform entlang des Nutgrundes erstrecken, wobei eine über die gesamte Nutlänge gleichmäßige Hubbewegung erreicht werden kann, indem in die keilförmigen, wellenförmigen oder angeschrägten Abschnitte gleichmäßigen Abständen mit korrespondieren Auslösetastern vorgesehen werden. Eine an dem Schlitten angeordnete Kopplungsvorrichtung kann dadurch unabhängig von einer aktuellen Längsachsenposition des Schlittens gelöst werden.

Alternativ oder zusätzlich kann in einem Bereich des zylindrischen Grundkörpers ein Segment ausgenommen sein, in dem die Führungsnut vorliegt. Das ausgenommene Segment bildet dabei eine Art Schiene bzw. eine Gleitfläche für den Schlitten, wobei der Schlitten einen T-förmigen bzw. mit einen Pilzquerschnitt haben kann. Die Gleitfläche kann zur weiteren Verbesserung der Ergonomieeigenschaften, insbesondere zur Verbesserung des Force-Feedback mit einer gleitfördernden Beschichtung oder Oberflächenstrukturierung versehen sein. Das ausgenommene Segment des zylindrischen Grundkörpers kann sich vorteilhaft in Richtung des zweiten Betätigungselements in der "Ruhestellung" des zweiten Betätigungselements bis hinter den Sperrring erstrecken, da durch Zusammenwirkung der Auswölbung des Sperrrings und der planen Gleitfläche des ausgenommenen Segments die Rotation des zweiten Drehelements begrenzt werden kann, indem die Auswölbung bei Erreichen des Freigabezustands auf der planen Gleitfläche anliegt und das zweite Betätigungselement nicht weiter verdreht werden kann. Der Querschnitt des zylindrischen Grundkörpers wird in dem Bereich des ausgenommenen Segments durch einen Kreisbogen und eine Kreissehne nach außen hin begrenzt, sodass man das ausgenommene Segment quasi als eine "Abplattung" in längsaxialer Richtung bezeichnen könnte.

Konzentrisch innerhalb des zweiten Betätigungselements kann eine Führungshülse ortsfest zum Grundkörper angeordnet sein, die wenigstens an einer dem Sperrzustand des zweiten Betätigungselements entsprechenden Winkelposition eine längsaxiale Führungsgasse aufweist, in der ein korrespondierendes Eingriffsmittel des zweiten Betätigungselements oder des Sperrrings geführt ist.

Die Führungsgasse der Führungshülse sorgt in Zusammenwirkung mit dem korrespondierenden Eingriffsmittel des zweiten Betätigungselements respektive des Sperrrings dafür, dass das zweite Betätigungselement während des längsaxialen Verschiebens zum Betätigen der Auslösevorrichtung nicht versehentlich verdreht werden kann. Dadurch kann das zweite Betätigungselement längsaxial verschoben werden, ohne dass der Anwender darauf achten müsste, es in der Rotationsposition zu halten.

Bei dem ersten Betätigungselement kann es sich insbesondere um ein Drehrad handeln, dass etwa über ein Bewegungsgewinde oder eine Kulissenführung mit dem Schlitten gekoppelt ist. Zur Erzeugung der Axialbewegung des Schlittens ist das Drehrad bezüglich der Längsachse des Grundkörpers verschiebefest zum Grundkörper angeordnet. Es kann auf seiner inneren Mantelfläche ein Bewegungsgewinde haben, das in Eingriff mit einem korrespondierenden Bewegungsgewindeabschnitt des Schlittens steht. Über eine Kulissenführung lässt sich ein nichtlineares Übersetzungsverhältnis zwischen der Drehbewegung des Drehrads und der Axialverschiebung des Schlittens erreichen, was zur Beeinflussung der Force-Feedback-Eigenschaften verwendet werden kann, insbesondere um ein nichtlineares Übersetzungsverhältnis eines operativ an die Handhabe gekoppelten Werkzeugs zu kompensieren.

Das erfindungsgemäße chirurgische Instrument weist in einer ersten Ausführungsform eine Handhabe auf, mit der ein Schaft gekoppelt ist, in dem längsaxial verschiebbar eine Betätigungsstange geführt ist, die mit einem längsaxial verschiebbaren Schlitten der Handhabe gekoppelt ist. An ein distales Ende des Schafts ist ein Werkzeugkopf angekoppelt, der mit der Betätigungsstange von einem offenen Zustand in einen geschlossenen Zustand überführt werden kann. Bei der Handhabe handelt es sich um eine erfindungsgemäße Handhabe, wie hierin offenbart.

Der Werkzeugkopf kann ein schneidendes Werkzeug, etwa eine Schere, oder ein greifendes Werkzeug, etwa eine Klemme, einen Nadelhalter, eine Zange, eine Pinzette oder ein aufhaltendes Werkzeug, etwa einen Retraktor (z. B. einen Ring- bzw. Flächenretraktor), tragen. Gewöhnlich werden solche Werkzeuge durch Zurückziehen der Betätigungsstange in den geschlossenen Zustand gebracht und durch Schieben der Betätigungsstange zum distalen Ende in den offenen Zustand. Bei bisher bekannten chirurgischen Instrumenten ist der Winkelbereich des Werkzeugs, der zwischen dem offenen Zustand und dem geschlossenen Zustand liegt, durch zwei Anschlagspositionen vorgegeben. Unter Verwendung der erfindungsgemäßen Handhabe hingegen kann neben den zwei Anschlagspositionen, die im Normalbetrieb verwendet werden, eine weitere Funktionsstellung des Werkzeugs erreicht werden, indem der Schlitten um den zusätzlichen Verschiebewegabschnitt verschoben wird, wodurch das Werkzeug in eine Überoffenposition gebracht werden kann. Die Überoffenposition wird nur selten benötigt, sie ist jedoch hilfreich beim Reinigen und / oder Zerlegen des chirurgischen Instruments, da die Handhabe in dieser Position arretiert werden kann, wodurch das Reinigen und / oder Zerlegen komfortabler und sicherer als bei bekannten chirurgischen Instrumenten möglich ist.

In einer weiteren Ausführungsform des chirurgischen Instruments kann der Schaft ein Hüllrohr aufweisen, das bevorzugt mit dem distalen Kopplungsabschnitt der Handhabe gekoppelt ist, insbesondere lösbar gekoppelt. Besonders bevorzugt weist das Hüllrohr ein Rastmittel auf, das insbesondere eine Rastvertiefung oder eine umlaufende Rastnut sein kann, das in Eingriff mit der Kopplungsvorrichtung des Grundkörpers steht. Alternativ oder zusätzlich kann die Betätigungsstange auch ein Rastmittel aufweisen, das bevorzugt eine Rastvertiefung oder eine umlaufende Rastnut sein kann, das mit der Kopplungsvorrichtung des Schlittens in Eingriff steht.

Das erfindungsgemäße Bedienverfahren für ein chirurgisches Instrument ermöglicht es, den Werkzeugkopf eines erfindungsgemäßen chirurgischen Instruments in eine Überoffenposition zu überführen. Dabei werden folgende Schritte ausgeführt:
- Drehen des zweiten Betätigungselements der Handhabe bis die Auswölbung des Sperrrings und Finger des Schlittens versetzt zueinander vorliegen, dadurch in den Freigabezustand Überführen des zweiten Betätigungselements
- dadurch Freigeben des Verschiebewegabschnitts des Schlittens,
- Betätigen des ersten Betätigungselements, dadurch Bewegen des Schlittens in Richtung des Sperrrings und Verschieben des Fingers bis zu einer Endposition hinter der Ringebene und dadurch mittelbar Überführen des Werkzeugkopfes in die Überoffenposition.

Wenn das Verfahren mit einer Ausführungsform des chirurgischen Instruments ausgeführt wird, dessen Grundkörper die segmentförmige Ausnehmung aufweist, wird der Endwinkel der Verdrehung des zweiten Betätigungselements vorteilhaft durch Anschlagen der Auswölbung des Sperrrings auf der Gleitfläche vorgegeben. Die Freigabeposition und die Sperrposition können auch "blind" erreicht werden; das Verfahren kann intuitiv ohne lange Einlernzeit ausgeführt werden. Ferner ist das Verfahren auch fehlersicher zu bedienen, da der Verschiebewegabschnitt des Schlittens erst nach einem aktiven Benutzereingriff, nämlich dem Verdrehen des zweiten Betätigungselements freigegeben wird.

Schließlich umfasst das erfindungsgemäße Zerlegeverfahren für ein erfindungsgemäßes chirurgisches Instruments die folgenden Schritte:
- Drehen des zweiten Betätigungselements der Handhabe bis die Auswölbung des Sperrrings und die Nase des Grundkörpers versetzt zueinander vorliegen, dadurch überführen des zweiten Betätigungselements in den Freigabezustand,
- dadurch Freigeben eines Durchtritts des Sperrrings für die Nase des Grundkörpers der Handhabe,
- Verschieben des zweiten Betätigungselements in einer zu der Nase weisenden Richtung, dadurch Mitbewegen und in Auslösestellung Bringen der Auslösevorrichtung, dabei Erzeugen einer Hubbewegung der Auslösevorrichtung in einer Richtung normal zu der Bewegungsrichtung des zweiten Betätigungselements,
- Übertragen der Hubbewegung der Auslösevorrichtung auf die Kopplungsvorrichtungen des Schlittens und des Grundkörpers, dadurch Betätigen der Kopplungsvorrichtungen und Lösen des Eingriffs des Rastmittels des Hüllrohrs des Schafts mit der Kopplungsvorrichtung des Grundkörpers und des Rastmittels der Betätigungsstange des Schafts mit der Kopplungsvorrichtung des Schlittens,
- gemeinsam Trennen des Hüllrohrs und der Betätigungsstange von der Handhabe.

Das Zerlegeverfahren kann sehr ergonomisch ausgeführt werden, da beide Rastmittel, das des Hüllrohrs und das der Betätigungsstange gleichzeitig durch längsaxiales Verschieben der zweiten Betätigungsvorrichtung ausgelöst werden. Es ist hierzu nur eine Hand nötig, während die andere Hand zum Halten der Handhabe oder zum Abnehmen des Instrumentenschafts frei bleibt.

Diese und weitere Vorteile werden durch die nachfolgende Beschreibung unter Bezug auf die begleitenden Figuren dargelegt. Der Bezug auf die Figuren in der Beschreibung dient der Unterstützung der Beschreibung und dem erleichterten Verständnis des Gegenstands. Gegenstände oder Teile von Gegenständen, die im Wesentlichen gleich oder ähnlich sind, können mit denselben Bezugszeichen versehen sein. Die Figuren sind lediglich schematische Darstellungen von Ausführungsbeispielen der Erfindung.

Es zeigen:
- Fig. 1a: eine perspektivische Teilansicht der Handhabe mit dem Schlitten an seiner proximalen Anschlagposition,
- Fig. 1b: eine Draufsicht auf einen Teil der Handhabe,
- Fig. 2a: eine perspektivische Teilansicht der Handhabe mit dem Schlitten an seiner distalen Anschlagposition,
- Fig. 2b: eine Draufsicht auf einen Teil der Handhabe,
- Fig. 3a: eine perspektivische Teilansicht der Handhabe mit dem Schlitten an seiner distalen Anschlagposition und mit dem zweiten Betätigungselement im Freigabezustand,
- Fig. 4a: eine perspektivische Teilansicht der Handhabe mit dem zweiten Betätigungselement im Freigabezustand und dem Schlitten distal hinter seiner distalen Anschlagposition,
- Fig. 4b: eine Draufsicht auf einen Teil der Handhabe,
- Fig. 5a: eine perspektivische Teilansicht der Handhabe mit dem zweiten Betätigungselement im Freigabezustand und dem Schlitten um den Verschiebewegabschnitt distal hinter seiner distalen Anschlagposition,
- Fig. 5b: eine Draufsicht auf einen Teil der Handhabe,
- Fig. 6: eine Längsschnittansicht eines Teils der Handhabe,
- Fig. 7: eine perspektivische Ansicht der Handhabe.

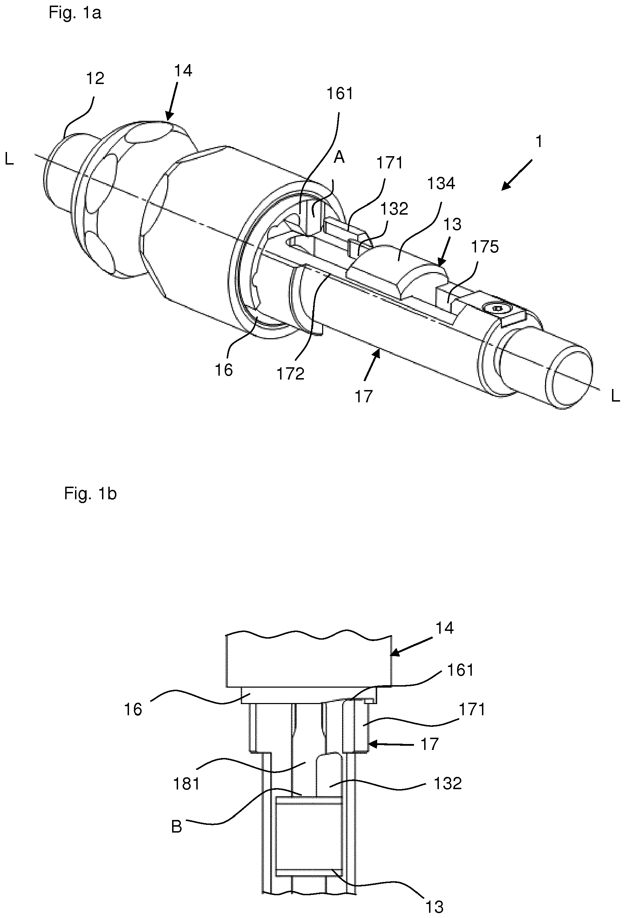

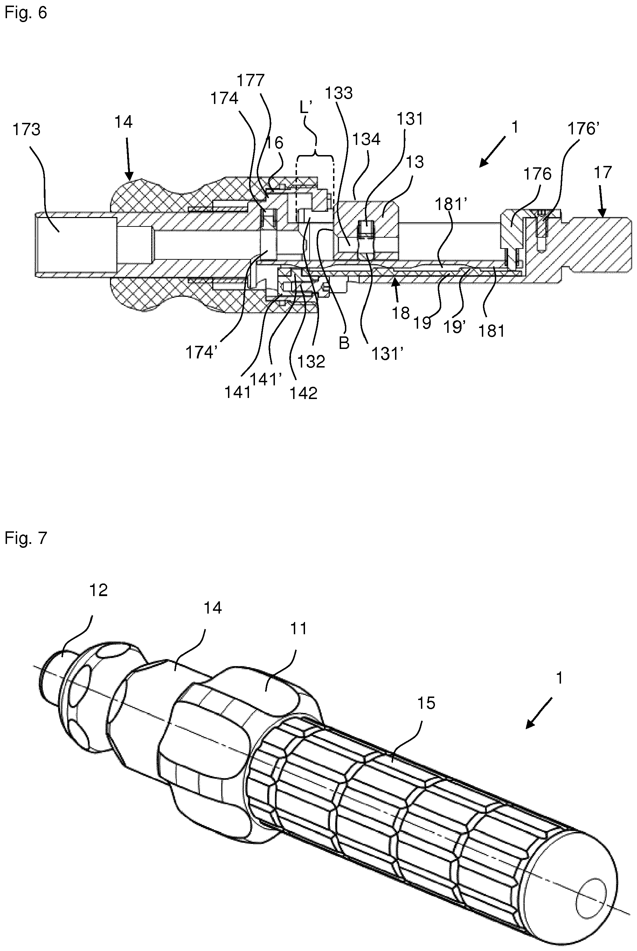

In Fig. 1a ist eine erfindungemäße Handhabe 1 in einer perspektivischen Teilansicht dargestellt, wobei der Griffteil 15 (siehe Fig. 7) zur besseren Sichtbarkeit der Bauteile unter dem Griffteil 15 ausgeblendet ist. Die Handhabe 1 weist einen länglichen bzw. zylindrischen Grundkörper 17 auf, der einen entlang seiner Längsachse L veränderlichen Querschnitt hat. Das in der Figur unten rechts liegende Ende des Grundkörpers ist das proximale Ende und das nach oben links weisende Ende der distale Kopplungsabschnitt 12, an dem ein (Instrumenten-) Schaft angekoppelt werden kann, der üblicherweise ein Hüllrohr und eine darin beweglich geführte Betätigungsstange hat.

Das Hüllrohr wird ortsfest mit dem Grundkörper 17 verbunden, während die Betätigungsstange dazu vorgesehen ist, mit dem längsaxial beweglichen Schlitten 13 verbunden zu werden. Der Schlitten 13 ist in einer Führungsnut 175, die sich entlang der Längsachse L des Grundkörpers 17 erstreckt, geführt und hat einen T-förmigen Querschnitt, während der Antrieb des Schlittens 13 über ein Bewegungsgewinde 134 auf seiner Mantelfläche realisiert ist, das in Eingriff mit einem Drehrad 11 als erstem Betätigungselement 11 (siehe Fig. 7) gebracht werden kann.

Im Bereich der Führungsnut 175 weist der Grundkörper 17 eine Abplattung auf, die durch Ausnehmung eines Zylindersegments aus dem Grundkörper 17 gebildet ist. Diese Ausnehmung oder Abplattung hat eine plane Fläche, die als Gleitfläche 172 für die beiden Schenkel des T-förmigen Schlittens 13 dient. Der Schlitten 13 kann von seiner in der Fig. 1a gezeigten distalen Anschlagstellung in eine proximale Position verfahren werden, wobei an einen distalen Anschlag 161 zunächst der Finger 132, der sich exzentrisch zur Längsachse des Grundkörpers 17 von einer nach distal weisenden Stirnfläche B (siehe Fig. 6) des Schlittens 13 erstreckt, zum Anliegen kommt. Die Handhabe 1 weist ferner ein zweites Betätigungselement 14 auf, das distal benachbart zum Drehrad 11, das hier nicht gezeigt ist (siehe Fig. 7) angeordnet ist. Das zweite Betätigungselement 14 hat zwei Bewegungsfreiheitsgrade: Es kann um die Längsachse L des Grundkörpers 17 rotiert werden und es kann längsaxial verschoben werden. In der gezeigten Stellung ist der längsaxiale Verschiebefreiheitsgrad jedoch dadurch gesperrt, dass eine Nase 171 die ortsfest am Grundkörper 17 angeordnet ist und einen hakenförmigen Querschnitt hat, der einen Hinterschnitt bildet, einen Anschlag bereitstellt. Das zweite Betätigungselement 14 ist innenliegend drehfest mit einem Sperrring 16 verbunden, der eine nach radial innen weisende Auswölbung 161 hat, die wiederum eine Sperrfläche A enthält, die zum Sperren der längsaxialen Bewegung an der Nase 171 anliegt.

Die Draufsicht in einer Richtung normal zur Gleitfläche 172 der Fig. 1b zeigt den Bereich um das proximale Ende des zweiten Betätigungselements 14 und den Schlitten 13, wobei am Grund der Führungsnut 175 ein Auslöseband 181 zu erkennen ist, das in einer aus der Bildebene heraus weisenden Richtung verfahrbar ist, um den Eingriff der Betätigungsstange mit dem Schlitten 13 zu lösen, der über eine lösbare Kopplungsvorrichtung auf der der Ansicht abgewandten Seite des Schlittens realisiert wird, die in Fig. 6 gut zu erkennen ist.

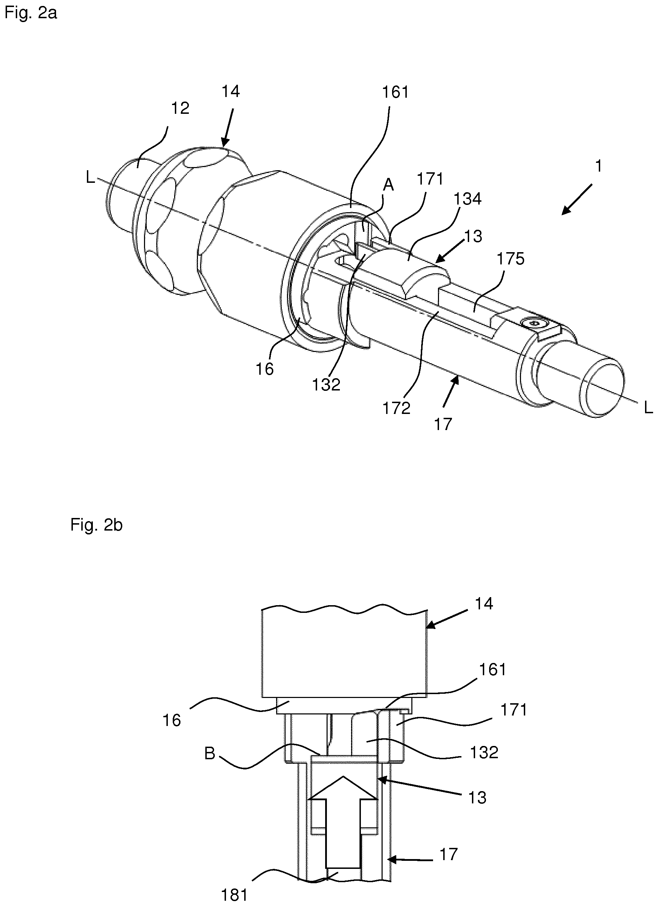

Der Finger 132 des Schlittens 13 und die Auswölbung sind so positioniert und hinsichtlich ihrer Abmessungen aufeinander abgestimmt, dass der Finger 132, wenn der Schlitten 13 an seinen distalen Anschlag gefahren ist, an der Auswölbung 161 anliegt, was in Fig. 2a und Fig. 2b gezeigt ist. Die Fläche A der Auswölbung 161 des Sperrrings 16 versperrt den weiteren Verschiebeweg nach distal. Der Verschiebeweg zwischen dem proximalen Anschlag (siehe Fig. 1a und Fig. 1b) und dem distalen Anschlag entspricht dabei dem Weg, der im Normalbetrieb für die Bedienung eines chirurgischen Werkzeugs, etwa einer Zange, Pinzette oder Schere verwendet wird, während ein darüber hinaus gehender Verschiebeweg des Schlittens 13 gesperrt bleibt.

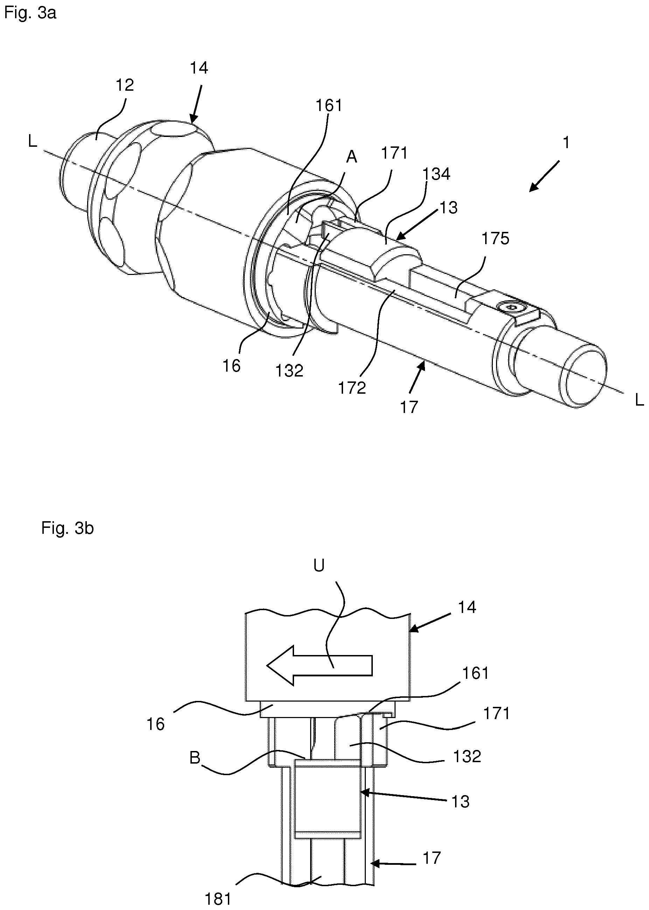

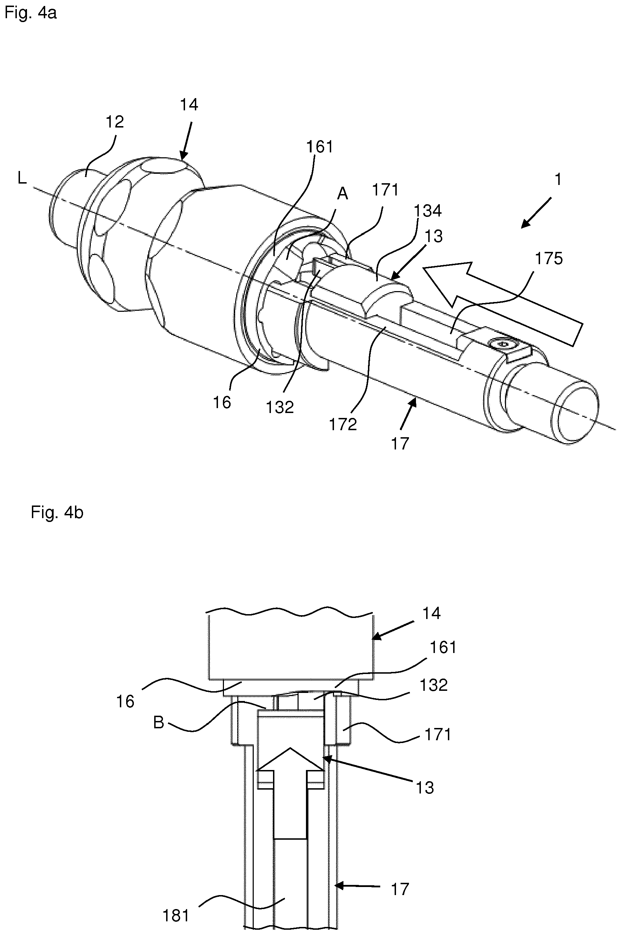

Soll nun ein weiterer Verschiebewegabschnitt L' (siehe Fig. 6) des Schlittens 13 in distale Richtungfreigegeben werden, wird das zweite Betätigungselement 14 in die Drehrichtung U (in der Zeichnung nach links) rotiert, wobei die Auswölbung 161 des Sperrrings zusammen mit dem zweiten Betätigungselement verdreht wird. Der Endzustand der Verdrehung ist in den Fig. 3a und Fig. 3b dargestellt. Die Auswölbung 161 liegt dann auf der Gleitfläche 172 des Abschnitts mit der zylindersegmentförmigen Ausnehmung des Grundkörpers 17 an und sperrt ein weiteres Verdrehen nach links. Der Finger 132 liegt nun nicht mehr auf der Auswölbung 161 an und kann weiter nach distal verfahren werden, bis die zum Sperrring 16 weisende Stirnfläche B des Schlittens 13 am Ende der Führungsnut 175 anliegt. Eine Position des Schlittens 13 kurz vor dem Anliegen der Stirnfläche B am Ende der Nut 175 ist in den Fig. 4a und Fig. 4b dargestellt, wobei die Bewegungsrichtung des Schlittens 13 entlang des nun freigegebenen Verschiebewegabschnitts L' (siehe Fig. 6) mit einem Pfeil gekennzeichnet ist. Zwischen der zum Sperrring 16 weisenden Stirnfläche B des Schlittens 13 und dem Sperrring 16 besteht jedoch noch ein Abstand. In der Draufsicht der Fig. 4b ist darüber hinaus eine Anschrägung der Auswölbung 161 zu erkennen, die in Drehrichtung U (siehe Fig. 3b) des zweiten Betätigungselements 14 respektive des Sperrrings nach proximal "ansteigt". Das zweite Betätigungselement 14 kann auch unter einer Belastung, die von dem Finger 132 des Sperrrings 13 auf die Auswölbung 161 im Sperrzustand (siehe Fig. 2b) ausgeübt wird und die zu einer Verspannung des Sperrrings 13 führt in seinen Freigabezustand verdreht werden, da die Anschrägung beim Verdrehen nach links einen Spalt zwischen der distalen Stirnfläche A des Fingers 132 und der Auswölbung freigibt, wodurch die Verspannung gelöst wird.

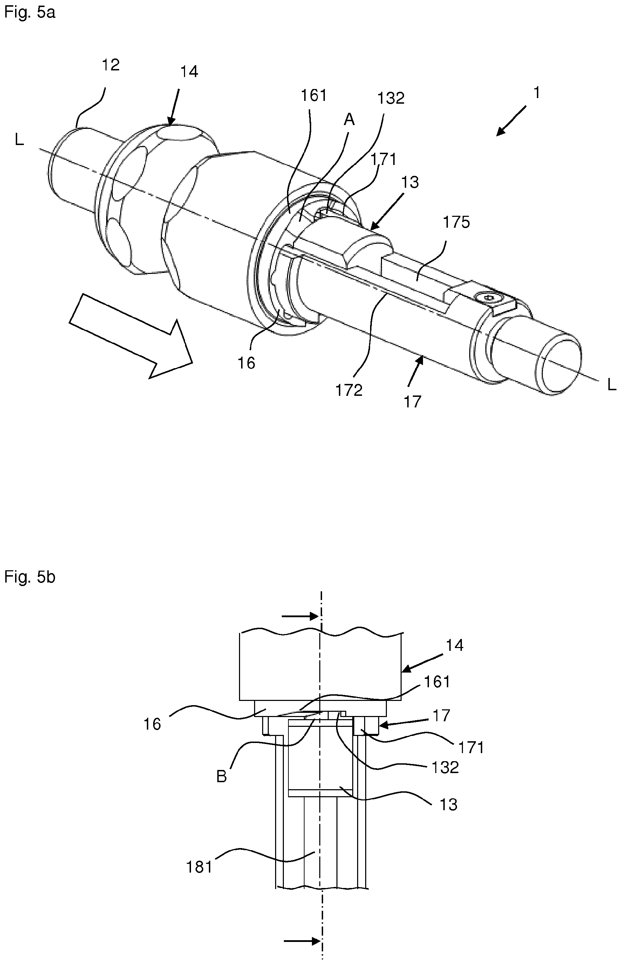

Der Querschnitt der Nase 171 ist hakenförmig, wobei der Übergang vom zylindrischen Grundkörper 17 zur Nase 171 tangential ausgeführt ist. Die Außenkontur des Querschnitts der Nase 171 stellt quasi eine knickfreie Fortführung der Außenkontur des Querschnitts des Grundkörpers 17 dar, wodurch es ermöglicht wird, dass die Nase 171 zwar im Sperrzustand des zweiten Betätigungselements 14 sperrend an der Auswölbung 161 des Sperrrings 16 anliegt, jedoch im Freigabezustand den Sperrring 16 nicht berührt, wodurch es möglich ist, das zweite Betätigungselement 14 und mit ihm auch den Sperrring 16 über die Nase 171 hinweg nach proximal zu bewegen, was in den Fig. 5a und 5b gezeigt ist, wobei die Fig. 5b eine Draufsicht der auch in der Fig. 5a gezeigten Funktionsstellung zeigt.

Der Zustand der Handhabe 1 mit partiell distal durch den Sperrring 16 geführtem Finger 132 ist auch im Längsschnitt der Fig. 6 gezeigt. Das zweite Betätigungselement 14 ist nach proximal verschoben, wobei ein Anschlag 177 des Grundkörpers 17 eine weitere Bewegung des zweiten Betätigungselements 14 nach proximal, d. h. in der Abbildung nach rechts, verhindert. Die Handhabe 1 ist dazu vorgesehen, mit einem Schaft gekoppelt zu werden, in dem eine Betätigungsstange verschiebbar geführt ist.

Zur Kopplung eines Hüllrohrs des Schafts ist in dem Grundkörper 17 eine Aufnahmebohrung 173 vorhanden, in die das Hüllrohr eingeführt wird. Das Hüllrohr wird mittels eines federbelasteten Rastschiebers 174 mit dem Grundkörper verrastet, während die Betätigungsstange (nicht gezeigt) bis zum Schlitten 13 und in dem Schlitten 13 in einer Aufnahmebohrung 133 geführt wird und mit dem federbelasteten Rastschieber 131 mit dem Schlitten 13 verrastet wird. Die beiden federbelasteten Rastschieber 131, 174 können durch einen gemeinsame Auslösevorrichtung 18 entsperrt werden, die ein ortsfestes Auslöseband 181 und ein bewegliches Auslöseband 19 aufweist. Das bewegliche Auslöseband 19 ist verschiebefest mit der zweiten Betätigungsvorrichtung 14 verbunden und wird, wenn diese nach proximal verschoben wird mit nach proximal bewegt. Die Auslöserippen 19' des beweglichen Auslösebands 19 gleiten dabei auf wellenförmigen Abschnitten 181' des ortsfesten Auslösebands 181 ab, wobei eine Hubbewegung senkrecht zur Bewegungsrichtung erzeugt wird, die auf Auslösepins 131', 174' der Rastschieber 131,174 des Schlittens 13 und des Grundkörpers 17 einwirkt, um die Verrastung zu lösen. Das bewegliche Auslöseband 19 ist mittels einer Anbindungsvorrichtung 141, die eine Anbindungsnase 141' trägt formschlüssig mit dem zweiten Betätigungselement 14 verbunden, wobei die Anbindungsvorrichtung 141 selbst mit dem zweiten Betätigungselement 14 verschraubt ist (siehe Schraube 142). Das ortsfeste Auslöseband 181 wird durch einen Festlegungsbügel 176, der mit der Schraube 176' mit dem Grundkörper 17 verbunden ist, in Position gehalten.

In Fig. 7 ist schließlich die komplette Handhabe 1 in perspektivischer Ansicht dargestellt, wobei auch der Griffteil 15 gezeigt ist, der in den Fig. 1a bis Fig. 6 ausgeblendet war. Ein Operateur kann das Drehrad 11 mit seinem Zeigefinger / Mittelfinger und Daumen ergonomisch betätigen, während ihn das zweite Betätigungselement 14 im Normalbetrieb nicht stört, da es distal des Drehrads 11 angeordnet ist. Soll das am Ende des Schafts angebrachte Werkzeug in die Überoffenstellung gebracht werden, kann die Handhabe 1 weiterhin mit einer Hand am Griffteil 15 gehalten werden, während mit der anderen Hand das zweite Betätigungselement 14 verdreht werden kann. Selbst eine Einhandbedienung ist vorteilhaft möglich. Auch das Trennen des Schafts von der Handhabe 1 ist ergonomisch möglich: Die Handhabe 1 wird mit einer Hand am Griffteil 15 gehalten während das Lösen der Verrastung des Hüllrohrs und der Betätigungsstange des Schafts gemeinsam durch Zurückziehen des zweiten Betätigungselements 14 erfolgt.

### Bezugszeichenliste

- 1: Handhabe
- 11: Erstes Betätigungselement, Drehrad
- 12: Distaler Kopplungsabschnitt
- 13: Verschiebbarer Schlitten
- 131: Rastvorrichtung, Rastschieber
- 131': Auslösepin
- 132: Finger des des Schlittens
- 133: Aufnahmebohrung des Schlittens
- 134: Bewegungsgewinde des Schlittens
- 14: Zweites Betätigungselement
- 141: Anbindungsvorrichtung des beweglichen Auslösebands
- 141': Anbindungsnase
- 142: Verschraubung
- 15: Griffteil
- 16: Sperrring
- 161: Auswölbung des Sperrrings
- 17: Grundkörper
- 171: Nase des Grundkörpers
- 172: Gleitfläche des Grundkörpers
- 173: Aufnahmebohrung des Grundkörpers
- 174: Rastvorrichtung des Grundkörpers
- 174': Auslösepin
- 175: Führungsnut des Grundkörpers
- 176: Festlegungsbügel des Auslösebands
- 176': Schraube
- 177: Anschlag des Grundkörpers
- 18: Auslösevorrichtung
- 181: Ortsfestes Auslöseband
- 181': keilförmiger, wellenförmiger oder angeschrägter Abschnitt des Auslösebands
- 19: Bewegliches Auslöseband
- 19': Auslöserippe
- A: Zum Sperrring weisende Stirnfläche des Fingers
- B: Stirnfläche des Schlittens
- L: Längsachse des Grundkörpers
- L': Verschiebewegabschnitt des Schlittens
- U: Drehrichtung des zweiten Betätigungselements

## Patentansprüche

1. Handhabe (1) für ein chirurgisches Instrument, umfassend
- einen distalen Kopplungsabschnitt (12) zur Kopplung mit einem Schaft, und
- einen länglichen Grundkörper (17), in dem ein Schlitten (13) längsaxial zwischen einer ersten Endposition und einer zweiten Endposition verschiebbar geführt ist,
- ein erstes Betätigungselement (11), das zum Axialbewegen des Schlittens (13) operativ mit diesem gekoppelt ist, wobei der Schlitten zum Längsaxialbewegen einer Betätigungsstange des Schafts des chirurgischen Instruments ausgebildet ist,
- ein zweites Betätigungselement (14) längsaxial benachbart zu dem ersten Betätigungselement (11) angeordnet ist, **dadurch gekennzeichnet, dass** das zweite Bedienelement um die Längsachse (L) des Grundkörpers (17) drehbar um diesen angeordnet ist, und
- das zweite Betätigungselement (14) zumindest an seinem dem ersten Betätigungselement (11) zugewandten Ende ringförmig ausgebildet ist und innenumfänglich einen Sperrring (16) drehfest trägt, dessen Innenwandung eine radial in den Sperrring (16) weisende Auswölbung (161) aufweist, die eine zumindest teilweise in der Ringebene liegende Fläche (A) hat,
- wobei der Schlitten (13) einen Finger (132) aufweist, der sich von einer zu dem Sperrring (16) weisenden Stirnfläche (B) des Schlittens (13) exzentrisch zu der Längsachse (L) des Grundkörpers (17) erstreckt,
- und wobei in einem Sperrzustand des zweites Betätigungselements (14) die Fläche (A) einen ersten Anschlag für den Schlitten (13) bereitstellt, indem der Finger (132)an der Fläche (A) zur Anlage kommt, und
- in einem Freigabezustand des zweiten Betätigungselements (14) der Finger (132) zu der Fläche (A) versetzt zu dem Sperrring (16) weist und einen Verschiebewegabschnitt (L') des Schlittens (13) freigibt.

2. Handhabe (1) nach Anspruch 1,
**dadurch gekennzeichnet, dass**
das zweite Betätigungselement (14) zumindest entlang eines Längsachsenabschnitts des Grundkörpers (17) verschiebbar ist.

3. Handhabe (1) nach Anspruch 1 oder 2,
**dadurch gekennzeichnet, dass**
- der Grundkörper (17) an seinem Außenumfang eine Nase (171) aufweist, die so positioniert ist, dass sie in dem Sperrzustand des zweiten Betätigungselements (14) an der Fläche (A) der Auswölbung (161) des Sperrrings (16) anliegt, wobei die Auswölbung (161) in Umfangsrichtung des Sperrrings (16) angeschrägt ist und in dem Freigabezustand des zweiten Betätigungselements (14) versetzt zu der Auswölbung (161) vorliegt,
wobei bevorzugt die Nase (171) einen hakenförmigen Querschnitt aufweist, der sich bevorzugt im Wesentlichen entlang des Außenumfangs des Grundkörpers (17), bevorzugt tangential, und entlang eines Abschnitts parallel zu der Längsachse (L) des Grundkörpers (17) erstreckt.

4. Handhabe (1) nach zumindest einem der Ansprüche 2 oder 3,
**dadurch gekennzeichnet, dass**
- der Schlitten (13) eine Kopplungsvorrichtung aufweist, bevorzugt eine Rastvorrichtung (131), besonders bevorzugt einen Rastschieber (131), am meisten bevorzugt einen federbelasteten Rastschieber(131), die mit der Betätigungsstange des Schafts lösbar koppelbar ist und
- der Grundkörper (17) eine Kopplungsvorrichtung aufweist, bevorzugt eine Rastvorrichtung (174), besonders bevorzugt einen Rastschieber (174), am meisten bevorzugt einen federbelasteten Rastschieber (174), der mit dem Hüllrohr des Schafts lösbar koppelbar ist.

5. Handhabe (1) nach Anspruch 4,
**dadurch gekennzeichnet, dass**
- das zweite Betätigungselement (14) operativ mit einer Auslösevorrichtung (18) gekoppelt ist, die sich parallel zu der Längsachse (L) des Grundkörpers (17) erstreckt und die zum Lösen der Kopplungsvorrichtungen des Grundkörpers (17) und des Schlittens (13) operativ mit den Kopplungsvorrichtungen des Grundkörpers (17) und des Schlittens (13) gekoppelt ist,
- wobei die Auslösevorrichtung (18) in dem Freigabezustand des zweiten Betätigungselements (14) zusammen mit dem zweiten Betätigungselement (14) längsaxial in eine Auslösestellung verschiebbar ist.

6. Handhabe (1) nach Anspruch 5,
**dadurch gekennzeichnet, dass**
- die Auslösevorrichtung (18) ein mit dem zweiten Betätigungselement (14) mitbewegbares erstes Teil und ein in Bezug auf die Längsachse des Grundkörpers ortsfestes zweites Teil aufweist, und
- der erste Teil und / oder der zweite Teil der Auslösevorrichtung (18) bevorzugt jeweils ein Auslöseband (181,19) umfasst, das zumindest einen im Längsschnitt keilförmigen, wellenförmigen oder angeschrägten Abschnitt aufweist, der eine Auslöserippe (19') des jeweils anderen Teils kontaktiert.

7. Handhabe (1) nach zumindest einem der Ansprüche 1 bis 6,
**dadurch gekennzeichnet, dass**
zwischen dem zweiten Betätigungselement (14) und dem Grundkörper (17)eine Rückstellvorrichtung, bevorzugt eine Feder, angeordnet ist, durch die das zweite Betätigungselement (14) rückstellbar ist.

8. Handhabe (1) nach zumindest einem der Ansprüche 1 bis 7,
**dadurch gekennzeichnet, dass**
der Grundkörper (17) eine längsaxiale Führungsnut (175) aufweist, in der der Schlitten (13) geführt ist, wobei an dem ersten Anschlag des Schlittens (13) ein Abstand zwischen der Stirnfläche (B) des Schlittens (13) und einem gegenüberliegenden Ende der Führungsnut (175) vorliegt, dessen Länge zumindest so groß ist wie die halbe Länge des Fingers(132).

9. Handhabe (1) nach Anspruch 8,
**dadurch gekennzeichnet, dass**
- in einem Bereich des zylindrischen Grundkörpers (17) ein Segment ausgenommen ist, in dem die Führungsnut (175) vorliegt, das eine Gleitfläche (172) für den Schlitten (13), der bevorzugt einen T-förmigen Querschnitt aufweist, ausbildet.

10. Handhabe (1) nach zumindest einem der Ansprüche 1 bis 9,
**dadurch gekennzeichnet, dass**
konzentrisch innerhalb des zweiten Betätigungselements (14) eine Führungshülse ortsfest zu dem Grundkörper (17) angeordnet ist, die zumindest an einer dem Sperrzustand des zweiten Betätigungselements (14) entsprechenden Winkelposition eine längsaxiale Führungsgasse aufweist, in der ein Eingriffsmittel des zweiten Betätigungselements (14) oder des Sperrrings (16) geführt ist.

11. Handhabe (1) nach zumindest einem der Ansprüche 1 bis 10,
**dadurch gekennzeichnet, dass**
das erste Betätigungselement (11) ein Drehrad (11) ist, das bevorzugt über ein Bewegungsgewinde oder eine Kulissenführung mit dem Schlitten (13) gekoppelt ist.

12. Chirurgisches Instrument, das eine Handhabe aufweist, mit der ein Instrumentenschaft gekoppelt ist, in dem eine Betätigungsstange längsaxial verschiebbar geführt ist,
- wobei die Betätigungsstange mit einem längsaxial verschiebbaren Schlitten (13) der Handhabe gekoppelt ist,
- und wobei der Schaft an einem distalen Schaftende mit einem Werkzeugkopf gekoppelt ist, der mittels der Betätigungsstange von einem Offenzustand in eine Geschlossenzustandüberführbar ist,
**dadurch gekennzeichnet, dass**
die Handhabe eine Handhabe (1) nach zumindest einem der Ansprüche 1 bis 11 ist.

13. Chirurgisches Instrument nach Anspruch 12,
**dadurch gekennzeichnet, dass**
- der Schaft ein Hüllrohr aufweist, das mit dem distalen Kopplungsabschnitt (12) der Handhabe gekoppelt ist, bevorzugt lösbar gekoppelt, wobei das Hüllrohr ein Rastmittel aufweist, bevorzugt eine Rastvertiefung oder umlaufende Rastnut, das in Eingriff mit der Kopplungsvorrichtung des Grundkörpers (17) steht, und / oder
- die Betätigungsstange ein Rastmittel aufweist, bevorzugt eine Rastvertiefung oder umlaufende Rastnut, das mit der Kopplungsvorrichtung des Schlittens (13) in Eingriff steht.

14. Bedienverfahren für ein chirurgisches Instrument nach Anspruch 12 oder 13,
**dadurch gekennzeichnet, dass**
der Werkzeugkopf in eine Überoffenposition überführbar ist,
umfassend die Schritte:
- Drehen des zweiten Betätigungselements (14) der Handhabe (1) bis die Auswölbung (161) des Sperrrings (16) und Finger (132) des Schlittens (13) versetzt zueinander vorliegen, dadurch in den Freigabezustand Überführen des zweiten Betätigungselements (14),
- dadurch Freigeben des Verschiebewegabschnitts (L') des Schlittens (13),
- Betätigen des ersten Betätigungselements (11), dadurch Bewegen des Schlittens (13) in Richtung des Sperrrings (16) und Verschieben des Fingers (132) bis zu einer Endposition hinter der Ringebene und dadurch mittelbar Überführen des Werkzeugkopfes in die Überoffenposition.

15. Zerlegeverfahren für ein chirurgisches Instrument nach Anspruch 13, wenn Anspruch 13 von den Ansprüchen 2, 3 und 5 abhängig ist, umfassend die Schritte:
- Drehen des zweiten Betätigungselements (14) der Handhabe (1) bis die Auswölbung (161) des Sperrrings (16) und die Nase (171) des Grundkörpers (17) versetzt zueinander vorliegen, dadurch überführen des zweiten Betätigungselements (14) in den Freigabezustand,
- dadurch Freigeben eines Durchtritts des Sperrrings (16) für die Nase (171) des Grundkörpers (17) der Handhabe (1),
- Verschieben des zweiten Betätigungselements (14) in einer zu der Nase (171) weisenden Richtung, dadurch Mitbewegen und in Auslösestellung Bringen der Auslösevorrichtung (18), dabei Erzeugen einer Hubbewegung der Auslösevorrichtung (18) in einer Richtung normal zu der Bewegungsrichtung des zweiten Betätigungselements (14),
- Übertragen der Hubbewegung der Auslösevorrichtung (18) auf die Kopplungsvorrichtungen des Schlittens (13) und des Grundkörpers (17), dadurch Betätigen der Kopplungsvorrichtungen und Lösen des Eingriffs des Rastmittels des Hüllrohrs des Schafts mit der Kopplungsvorrichtung des Grundkörpers (17) und des Rastmittels der Betätigungsstange des Schafts mit der Kopplungsvorrichtung des Schlittens (13),
- gemeinsam Trennen des Hüllrohrs und der Betätigungsstange von der Handhabe (1).

## Claims

1. Handle (1) for a surgical instrument, comprising
- a distal coupling portion (12) for coupling to a shank, and
- an elongate main body (17), in which a carriage (13) is guided movably along the longitudinal axis between a first end position and a second end position,
- a first actuation element (11), which is operatively coupled to the carriage (13) in order to move the latter axially, wherein the carriage is designed for the longitudinal axial movement of an actuation rod of the shank of the surgical instrument,
- a second actuation element (14) is arranged adjacent to the first actuation element (11) on the longitudinal axis, **characterized in that** the second actuation element is rotatable about the longitudinal axis (L) of the elongate main body (17), and
- the second actuation element (14) is annular, at least at its end directed toward the first actuation element (11), and carries on its inner circumference a rotationally fixed locking ring (16) whose inner wall has a protuberance (161) facing radially into the locking ring (16), said protuberance (161) having a surface (A) lying at least partially in the ring plane,
- wherein the carriage (13) has a finger (132) which extends, eccentrically with respect to the longitudinal axis (L) of the main body (17), from a front face (B) of the carriage (13) facing the locking ring (16),
- and wherein, in a locked state of the second actuation element (14), the surface (A) provides a first abutment for the carriage (13), since the finger (132) comes to bear on the surface (A), and
- in a release state of the second actuation element (14), the finger (132), offset with respect to the surface (A), faces the locking ring (16) and frees a range of travel (L') of the carriage (13).

2. Handle (1) according to Claim 1, **characterized in that** the second actuation element (14) is movable at least along a longitudinal axial portion of the main body (17).

3. Handle (1) according to Claim 1 or 2, **characterized in that**
- the main body (17) has, on its outer circumference, a lug (171) which is positioned such that, in the locked state of the second actuation element (14), it bears on the surface (A) of the protuberance (161) of the locking ring (16), wherein the protuberance (161) is beveled in the circumferential direction of the locking ring (16) and, in the release state of the second actuation element (14), it is offset with respect to the protuberance (161),
wherein the lug (171) preferably has a hook-shaped cross section that preferably extends substantially along the outer circumference of the main body (17), preferably tangentially, and along a portion parallel to the longitudinal axis (L) of the main body (17).

4. Handle (1) according to at least one of Claims 2 and 3, **characterized in that**
- the carriage (13) has a coupling device, preferably a locking device (131), particularly preferably a locking slide (131), most preferably a spring-loaded locking slide (131), which can be coupled releasably to the actuation rod of the shank, and
- the main body (17) has a coupling device, preferably a locking device (174), particularly preferably a locking slide (174), most preferably a spring-loaded locking slide (174), which can be coupled releasably to the jacket tube of the shank.

5. Handle (1) according to Claim 4, **characterized in that**
- the second actuation element (14) is operatively coupled to a trigger device (18), which extends parallel to the longitudinal axis (L) of the main body (17) and which, in order to release the coupling devices of the main body (17) and of the carriage (13), is operatively coupled to the coupling devices of the main body (17) and of the carriage (13),
- wherein the trigger device (18), in the release state of the second actuation element (14), is movable along the longitudinal axis together with the second actuation element (14) to a trigger position.

6. Handle (1) according to Claim 5, **characterized in that**
- the trigger device (18) has a first part, which can be entrained with the second actuation element (14), and a second part, which is stationary with respect to the longitudinal axis of the main body, and
- the first part and/or the second part of the trigger device (18) preferably comprise(s) a trigger band (181, 19), which has at least one wedge-shaped, undulating or beveled portion in longitudinal section, which contacts a trigger rib (19') of the respective other part.

7. Handle (1) according to at least one of Claims 1 to 6, **characterized in that** a resetting device, preferably a spring, is arranged between the second actuation element (14) and the main body (17), by means of which the second actuation element (14) can be reset.

8. Handle (1) according to at least one of Claims 1 to 7, **characterized in that** the main body (17) has a longitudinal axial guide groove (175) in which the carriage (13) is guided, wherein, at the first abutment of the carriage (13), there is a distance between the front face (B) of the carriage (13) and an opposite end of the guide groove (175), the length of which distance is at least as great as half the length of the finger (132).

9. Handle (1) according to Claim 8, **characterized in that**
- in an area of the cylindrical main body (17), a segment is recessed in which the guide groove (175) is present, which segment has a sliding surface (172) for the carriage (13), which preferably has a T-shaped cross section.

10. Handle (1) according to at least one of Claims 1 to 9, **characterized in that**, concentrically within the second actuation element (14), a guide sleeve is arranged stationary with respect to the main body (17) and, at least at an angle position corresponding to the locked state of the second actuation element (14), has a longitudinal axial guide track in which an engagement means of the second actuation element (14) or of the locking ring (16) is guided.

11. Handle (1) according to at least one of Claims 1 to 10, **characterized in that** the first actuation element (11) is a rotary wheel (11), which is preferably coupled to the carriage (13) via a movement thread or a slotted guide.

12. Surgical instrument having a handle to which is coupled an instrument shank in which an actuation rod is guided movably along the longitudinal axis,
- wherein the actuation rod is coupled to a longitudinally axially movable carriage (13) of the handle,
- and wherein the shank is coupled, at a distal end of the shank, to a tool head which can be transferred by means of the actuation rod from an open state to a closed state,
**characterized in that**
the handle is a handle (1) according to at least one of Claims 1 to 11.

13. Surgical instrument according to Claim 12, **characterized in that**
- the shank has a jacket tube which is coupled, preferably releasably coupled, to the distal coupling portion (12) of the handle, wherein the jacket tube has a locking means, preferably a locking indent or circumferential locking groove, which is in engagement with the coupling device of the main body (17), and/or
- the actuation rod has a locking means, preferably a locking indent or circumferential locking groove, which is in engagement with the coupling device of the carriage (13).

14. Operating method for a surgical instrument according to Claim 12 or 13, **characterized in that** the tool head can be transferred to a beyond open position, said method comprising the steps of:
- rotating the second actuation element (14) of the handle (1) until the protuberance (161) of the locking ring (16) and the finger (132) of the carriage (13) are offset with respect to each other, thereby transferring the second actuation element (14) to the release state,
- thereby releasing the range of travel (L') of the carriage (13),
- actuating the first actuation element (11), thereby moving the carriage (13) in the direction of the locking ring (16) and moving the finger (132) as far as an end position behind the ring plane, and thereby indirectly transferring the tool head to the beyond open position.

15. Dismantling method for a surgical instrument according to Claim 13, when Claim 13 is dependent on Claims 2, 3 and 5, said method comprising the steps of:
- rotating the second actuation element (14) of the handle (1) until the protuberance (161) of the locking ring (16) and the lug (171) of the main body (17) are offset with respect to each other, thereby transferring the second actuation element (14) to the release state,
- thereby freeing a passage of the locking ring (16) for the lug (171) of the main body (17) of the handle (1),
- moving the second actuation element (14) in a direction toward the lug (171), thereby entraining the trigger device (18) and bringing it to the trigger position, thus generating a lifting movement of the trigger device (18) in a direction perpendicular to the direction of movement of the second actuation element (14),
- transmitting the lifting movement of the trigger device (18) to the coupling devices of the carriage (13) and of the main body (17), thereby actuating the coupling devices and releasing the engagement of the locking means of the jacket tube of the shank with the coupling device of the main body (17) and of the locking means of the actuation rod of the shank with the coupling device of the carriage (13),
- jointly separating the jacket tube and the actuation rod from the handle (1).

## Revendications

1. Poignée (1) pour un instrument chirurgical, comprenant
- une portion d'accouplement distale (12) pour l'accouplement à un arbre, et
- un corps de base allongé (17) dans lequel un chariot (13) est guidé de manière déplaçable axialement en longueur entre une première position d'extrémité et une deuxième position d'extrémité,
- un premier élément d'actionnement (11) qui, pour le déplacement axial du chariot (13), est accouplé fonctionnellement à ce dernier, le chariot étant réalisé pour le déplacement axial en longueur d'une tige d'actionnement de l'arbre de l'instrument chirurgical,
- un deuxième élément d'actionnement (14) étant disposé axialement en longueur à côté du premier élément d'actionnement (11),
**caractérisée en ce que** le deuxième élément d'actionnement est disposé de manière à pouvoir tourner autour de l'axe longitudinal (L) du corps de base (17), autour de ce dernier, et
- le deuxième élément d'actionnement (14) est réalisé au moins au niveau de son extrémité tournée vers le premier élément d'actionnement (11) sous forme annulaire et porte, de manière solidaire en rotation, au niveau de la périphérie intérieure, une bague d'arrêt (16) dont la paroi interne présente un bombement (161) orienté radialement dans la bague d'arrêt (16), qui présente une surface (A) située au moins en partie dans le plan de la bague,
- le chariot (13) présentant un doigt (132) qui s'étend depuis une surface frontale (B) du chariot (13) tournée vers la bague d'arrêt (16) de manière excentrique vers l'axe longitudinal (L) du corps de base (17),
- et, dans un état de blocage du deuxième élément d'actionnement (14), la surface (A) fournissant une première butée pour le chariot (13), par le fait que le doigt (132) vient buter contre la surface (A), et
- dans un état de libération du deuxième élément d'actionnement (14), le doigt (132) est tourné vers la bague d'arrêt (16) de manière décalée par rapport à la surface (A) et libère une portion mobile en coulissement (L') du chariot (13).

2. Poignée (1) selon la revendication 1,
**caractérisée en ce que**
le deuxième élément d'actionnement (14) peut être déplacé au moins le long d'une portion d'axe longitudinal du corps de base (17).

3. Poignée (1) selon la revendication 1 ou 2,
**caractérisée en ce que**
- le corps de base (17) présente, au niveau de sa périphérie extérieure, un ergot (171) qui est positionné de telle sorte qu'il s'applique, dans l'état de blocage du deuxième élément d'actionnement (14), contre la surface (A) du bombement (161) de la bague d'arrêt (16), le bombement (161) étant biseauté dans la direction périphérique de la bague d'arrêt (16), et de telle sorte qu'il soit décalé par rapport au bombement (161) dans l'état de libération du deuxième élément d'actionnement (14),
l'ergot (171) présentant de préférence une section transversale en forme de crochet qui s'étend de préférence essentiellement le long de la périphérie extérieure du corps de base (17), de préférence tangentiellement, et le long d'une portion parallèlement à l'axe longitudinal (L) du corps de base (17).

4. Poignée (1) selon au moins l'une quelconque des revendications 2 ou 3,
**caractérisée en ce que**
- le chariot (13) présente un dispositif d'accouplement, de préférence un dispositif d'encliquetage (131), particulièrement préférablement un coulisseau d'encliquetage (131), le plus préférablement un coulisseau d'encliquetage sollicité par ressort (131) qui peut être accouplé de manière détachable à la tige d'actionnement de l'arbre et
- le corps de base (17) présente un dispositif d'accouplement, de préférence un dispositif d'encliquetage (174), particulièrement préférablement un coulisseau d'encliquetage (174), le plus préférablement un coulisseau d'encliquetage sollicité par ressort (174) qui peut être accouplé de manière détachable au tube d'enveloppe de l'arbre.

5. Poignée (1) selon la revendication 4,
**caractérisée en ce que**
- le deuxième élément d'actionnement (14) est accouplé fonctionnellement à un dispositif de déclenchement (18) qui s'étend parallèlement à l'axe longitudinal (L) du corps de base (17) et, pour le desserrage des dispositifs d'accouplement du corps de base (17) et du chariot (13), est accouplé fonctionnellement aux dispositifs d'accouplement du corps de base (17) et du chariot (13),
- le dispositif de déclenchement (18) pouvant être déplacé axialement longitudinalement conjointement avec le deuxième élément d'actionnement (14) dans une position de déclenchement dans l'état de libération du deuxième élément d'actionnement (14).

6. Poignée (1) selon la revendication 5,
**caractérisée en ce que**
- le dispositif de déclenchement (18) présente une première partie déplacée en même temps que le deuxième élément d'actionnement (14) et une deuxième partie fixée par rapport à l'axe longitudinal du corps de base, et
- la première partie et/ou la deuxième partie du dispositif de déclenchement (18) comprennent de préférence chacune une bande de déclenchement (181, 19) qui présente au moins une portion en forme de clavette, de forme ondulée ou biseautée en coupe longitudinale, qui vient en contact avec une nervure de déclenchement (19') de l'autre partie respective.

7. Poignée (1) selon au moins l'une quelconque des revendications 1 à 6,
**caractérisée en ce**
**qu'**un dispositif de rappel, de préférence un ressort, est disposé entre le deuxième élément d'actionnement (14) et le corps de base (17), par le biais duquel le deuxième élément d'actionnement (14) peut être rappelé.

8. Poignée (1) selon au moins l'une quelconque des revendications 1 à 7,
**caractérisée en ce que**
le corps de base (17) présente une rainure de guidage axiale longitudinale (175) dans laquelle est guidé le chariot (13), une distance entre la surface frontale (B) du chariot (13) et une extrémité opposée de la rainure de guidage (175) étant prévue au niveau de la première butée du chariot (13), dont la longueur est au moins identique à la moitié de la longueur du doigt (132).

9. Poignée (1) selon la revendication 8,
**caractérisée en ce que**
- dans une région du corps de base cylindrique (17), est enlevé un segment dans lequel est réalisée la rainure de guidage (175), qui constitue une surface de glissement (172) pour le chariot (13) qui présente de préférence une section transversale en forme de T.

10. Poignée (1) selon au moins l'une quelconque des revendications 1 à 9,
**caractérisée en ce**
**qu'**une douille de guidage est disposée fixement par rapport au corps de base (17) concentriquement à l'intérieur du deuxième élément d'actionnement (14), laquelle présente, au moins au niveau d'une position angulaire correspondant à l'état de blocage du deuxième élément d'actionnement (14), une voie de guidage axiale longitudinale dans laquelle est guidé un moyen d'engagement du deuxième élément d'actionnement (14) ou de la bague d'arrêt (16).

11. Poignée (1) selon au moins l'une quelconque des revendications 1 à 10,
**caractérisée en ce que**
le premier élément d'actionnement (11) est un bouton rotatif (11) qui est de préférence accouplé au chariot (13) par le biais d'un filetage de déplacement ou d'un guide à coulisse.

12. Instrument chirurgical qui présente une poignée avec laquelle un arbre d'instruments est accouplé, dans lequel une tige d'actionnement est guidée de manière déplaçable axialement longitudinalement,
- la tige d'actionnement étant accouplée à un chariot (13) de la poignée, déplaçable axialement longitudinalement,
- et l'arbre étant accouplé au niveau d'une extrémité d'arbre distale à une tête d'outil qui peut être transférée au moyen de la tige d'actionnement d'un état ouvert dans un état fermé,
**caractérisé en ce que**
la poignée est une poignée (1) selon au moins l'une quelconque des revendications 1 à 11.

13. Instrument chirurgical selon la revendication 12,
**caractérisé en ce que**
- l'arbre présente un tube d'enveloppe qui est accouplé à la portion d'accouplement distale (12) de la poignée, de préférence qui est accouplé de manière détachable à celle-ci, le tube d'enveloppe présentant un moyen d'encliquetage, de préférence un renfoncement d'encliquetage ou une rainure d'encliquetage périphérique qui est en prise avec le dispositif d'accouplement du corps de base (17), et/ou
- la tige d'actionnement présente un moyen d'encliquetage, de préférence un renfoncement d'encliquetage ou une rainure d'encliquetage périphérique qui est en prise avec le dispositif d'accouplement du chariot (13).

14. Procédé de commande pour un instrument chirurgical selon la revendication 12 ou 13,
**caractérisé en ce que**
la tête d'outil peut être transférée dans une position d'ouverture accrue, comprenant les étapes suivantes :
- rotation du deuxième élément d'actionnement (14) de la poignée (1) jusqu'à ce que le bombement (161) de la bague d'arrêt (16) et le doigt (132) du chariot (13) soient situés de manière décalée l'un par rapport à l'autre, et de ce fait transfert dans l'état de libération du deuxième élément d'actionnement (14),
- de ce fait libération de la portion mobile en coulissement (L') du chariot (13),
- actionnement du premier élément d'actionnement (11), et de ce fait déplacement du chariot (13) dans la direction de la bague d'arrêt (16) et déplacement du doigt (132) jusqu'à une position d'extrémité derrière le plan de la bague et de ce fait transfert indirect de la tête d'outil dans la position d'ouverture accrue.

15. Procédé de démontage pour un instrument chirurgical selon la revendication 13, lorsque la revendication 13 dépend des revendications 2, 3 et 5, comprenant les étapes suivantes :
- rotation du deuxième élément d'actionnement (14) de la poignée (1) jusqu'à ce que le bombement (161) de la bague d'arrêt (16) et l'ergot (171) du corps de base (17) soient décalés l'un par rapport à l'autre, et de ce fait transfert du deuxième élément d'actionnement (14) dans l'état de libération,
- et de ce fait libération d'un passage de la bague d'arrêt (16) pour l'ergot (171) du corps de base (17) de la poignée (1),
- déplacement du deuxième élément d'actionnement (14) dans une direction tournée vers l'ergot (171), et de ce fait entraînement et déplacement dans la position de déclenchement du dispositif de déclenchement (18), en produisant ainsi un mouvement de course du dispositif de déclenchement (18) dans une direction perpendiculaire à la direction de déplacement du deuxième élément d'actionnement (14),
- transfert du mouvement de course du dispositif de déclenchement (18) aux dispositifs d'accouplement du chariot (13) et du corps de base (17), et de ce fait actionnement des dispositifs d'accouplement et libération de l'engagement du moyen d'encliquetage du tube d'enveloppe de l'arbre avec le dispositif d'accouplement du corps de base (17) et du moyen d'encliquetage de la tige d'actionnement de l'arbre avec le dispositif d'accouplement du chariot (13),
- séparation conjointe du tube d'enveloppe et de la tige d'actionnement de la poignée (1).
